# EUROPEAN PATENT APPLICATION

(11) **EP 3 621 086 A1**
(43) Date of publication of application: **11.03.2020**
(21) Application number: 18192896.1
(22) Date of filing: 06.09.2018
(51) Int. Cl.: G16H 40/63

(54) **AUGMENTED REALITY USER GUIDANCE DURING EXAMINATIONS OR INTERVENTIONAL PROCEDURES**

(71) Applicant: Koninklijke Philips N.V., 5656 AE Eindhoven (NL)
(72) Inventor: DEN HARTOG, Markus Johannes Harmen, 5656 AE Eindhoven (NL); OLIVAN BESCOS, Javier, 5656 AE Eindhoven (NL); ELENBAAS, Thijs, 5656 AE Eindhoven (NL); VAN DER STERREN, William Edward Peter, 5656 AE Eindhoven (NL); RUIJTERS, Daniël Simon Anna, 5656 AE Eindhoven (NL)
(74) Representative: de Haan, Poul Erik

(57) **Abstract**

The present invention relates to guidance during examinations or interventional procedures. In order to facilitate information provision in a medical environment such as an operation room or cathlab, an augmented reality display device (10) for medical equipment is provided that comprises a data input unit (12), a processing unit (20) and a display unit (22). The data input unit is configured to receive displayed operation parameters (14) of at least one medical appliance. The data input unit is also configured to receive relative location information (16) of at least one medical appliance in relation to the display unit and a viewing direction information (18) of the user. The processing unit is configured to detect if at least one of the medical appliances is in the user's field of view based on the relative location information and the viewing direction information. The processing unit is further configured to generate display data based on the operation parameters of the detected medical appliance. The display unit is configured to project information (24) related to the operation parameters of the medical appliance visible for the user based on the display data if the medical appliance is in the user's field of view. The display unit is configured to display the information as visible representation overlaid to reality.

## Description

### FIELD OF THE INVENTION

The present invention relates to guidance during examinations or interventional procedures, and relates in particular to an augmented reality display device, to an information system for medical equipment, to a method for providing operation parameters of medical equipment, as well as to a computer program element and to a computer readable medium.

### BACKGROUND OF THE INVENTION

During medical examinations or interventional procedures, such as in a catheter laboratory (cathlab), or intervention or operation room in a hospital, a variety of different equipment is used. As an example, the equipment may belong to the groups of treatment devices, diagnostic devices, imaging devices and other support devices. A large variety of data is provided, for example, to the clinical staff like surgeons, technical equipment operators, nurses and others. Central and auxiliary displays are provided to present the information before, during and after an examination or intervention. Some a part of the information is shown on display elements on the devices themselves, like a current flow of injected contrast agent. Further, for providing additional information, also so-called augmented reality is used. For example, WO 2018 052966 A1 describes augmented reality surgical technique guidance. In an example, non-visual information is presented to the user by augmented reality like information about the inside of an object. However, it has been shown that it is sometimes cumbersome for a user to identify or observe the required information.

### SUMMARY OF THE INVENTION

There may thus be a need to facilitate information provision in a medical environment such as an operation room or cathlab.

The object of the present invention is solved by the subject-matter of the independent claims; further embodiments are incorporated in the dependent claims. It should be noted that the following described aspects of the invention apply also for the augmented reality display device, for the information system for medical equipment and for the method for providing operation parameters of medical equipment.

According to the present invention, an augmented reality display device for medical equipment is provided. The device comprises a data input unit, a processing unit and a display unit. The data input unit is configured to receive displayed operation parameters of at least one medical appliance, and to receive relative location information of at least one medical appliance in relation to the display unit and a viewing direction information of the user. The processing unit is configured to detect if at least one of the medical appliances is in the user's field of view based on the relative location information and the viewing direction information, and to generate display data based on the operation parameters of the detected medical appliance. Further, the display unit is configured to project information related to the operation parameters of the medical appliance visible for the user based on the display data if the medical appliance is in the user's field of view. The display unit is configured to display the information as visible representation overlaid to reality.

According to an example, the processing unit is configured to generate duplicate display data based on the operation parameters of the detected medical appliance. The display unit is configured to project a duplication of at least a part of the operation parameters of the medical appliance visible for the user based on the duplicate display data if the medical appliance is in the user's field of view. The display unit is also configured to display the duplication of the operation parameters as visible representation overlaid to reality.

The visible representation is overlaid to the reality as seen by the particular user. The duplication of the operation parameters is thus only visible for the user.

By providing duplicate information an augmented reality, it is ensured that all information can be provided in a facilitated and targeted manner. Thus, a physician, or other user, is permanently informed of the settings and status of the devices used, in particular procedure-critical devices. The physician can stay focused on his task and does not need to ask other staff members like a technician or nurse to relay the information to him, when the device display is out of sight or hard to read. The physician also does not need to physically move from his/her location to get a better view of the device. Continuous monitoring, if required, is thus made possible. The workflow is thus further optimized. In extreme cases, it is ensured that correct settings can be checked and used and that a clinician is acting upon accurate information. To provide the additional, i.e. duplicate information only when the device is in the user's field of view ensures that the field of view does not get cluttered with information that is currently not of interest, which is taken from the assumption that only that information is of particular interest that belongs to devices that the user is looking for, i.e. when the user is looking in the direction of these devices.

The duplication of the operation parameters of the medical appliance is only visible for the particular user using the augmented reality display device. Hence, although the information is provided in a duplicated and thus redundant manner, separate displays are not used which would clutter the space in the room, in particular the space around the object support.

According to an example, for the relative location information, the data input unit is configured to receive location information of the at least one medical appliance, position information of the display unit and orientation information of the user. Further, the processing unit is configured to derive the relative location information with respect to the user from this information.

According to an example, the visible representation is displayed overlaid to reality depending on the presence of the medical appliance in the user's field of view. Further, in addition or alternatively, the display unit is configured to project the visible representation in the field of view in vicinity of the medical appliance comprising at least one of the group of i) next to the medical appliance and ii) overlaid to the medical appliance. Still further, also in addition or alternatively, the display unit is configured to also project a device indicator for pointing to or highlighting at least one medical appliance for which the visible representation is presented. According to an example, the data input unit is configured to receive user settings comprising at least information about an assignment to at least one of a plurality of pre-determined user categories. Further, the processing unit is configured to adapt the display data based on the user settings.

According to an example, the display unit is a head mounted display that comprises a display device which is configured i) to allow the user to look through for at least a part of the user's field of view, and ii) to provide the visible representation on the display element within the user's field of view.

According to an example, the processing unit is configured to temporarily modify the visible duplication when at least one of the displayed operation parameters reaches a predetermined threshold. Further, the display unit is configured to project the modified visible duplication as an interactive signal to the user.

Hence, an intuitive alarm-type of signal is provided.

According to the present invention, also an information system for medical equipment is provided. The system comprises at least one augmented reality display device according to one of the examples described above. The system also comprises at least one medical appliance with displayed operation parameters and a displayed operation parameters transmitting arrangement. The displayed operation parameters transmitting arrangement is configured to provide information about the displayed operation parameters from the at least one medical appliance to the data input unit of the at least one augmented reality display device.

According to the present invention, also a method for providing operation parameters of medical equipment is provided. The method comprises the following steps:
a) receiving displayed operation parameters of at least one medical appliance;
b) receiving relative location information of at least one medical appliance in relation to a display unit of an augmented reality display device for medical equipment and a viewing direction information of the user;
c) detecting if at least one of the medical appliances is in the user's field of view based on the relative location information and the viewing direction information;
d) generating display data based on the operation parameters of the detected medical appliance; and
e) projecting information related to the operation parameters of the medical appliance for the user based on the duplicate display data if the medical appliance is in the user's field of view, wherein the information is displayed as visible representation overlaid to reality of a live image stream as augmented reality.

According to an aspect, a duplication of displayed information is provided to a user with an augmented reality device such as a head mounted display. By providing the display unit, a setting of a device can be discerned, using an application programming interface (API), a screen capture or any other technique, the information can be displayed above or next to the device. The location itself of the device can be discovered using e.g. RFID tagging, depth camera tracking, optical camera tracking or other tracking location systems. The tracking device may be integrated with the augmented reality device, or may be a separate device. Since the content provided on the display unit is artificially rendered, other features could be provided in addition to the duplicated display content. In an example, the duplicated information is provided such that the information is always positioned above the respective device, and also facing the viewer. Providing the duplicated display information, can also be referred to as rendering remote device display information in augmented reality.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic view of an augmented reality display device for medical equipment.
Fig. 2 shows a further example of an augmented reality display device.
Fig. 3 shows another example of an augmented reality display device where the display is provided as a head mounted display.
Fig. 4 shows a still further example of an augmented reality display device.
Fig. 5 shows an information system for medical equipment with an example of the augmented reality display device.
Fig. 6 shows basic steps of an example of a method for providing operation parameters of medical equipment.

### DETAILED DESCRIPTION OF EMBODIMENTS

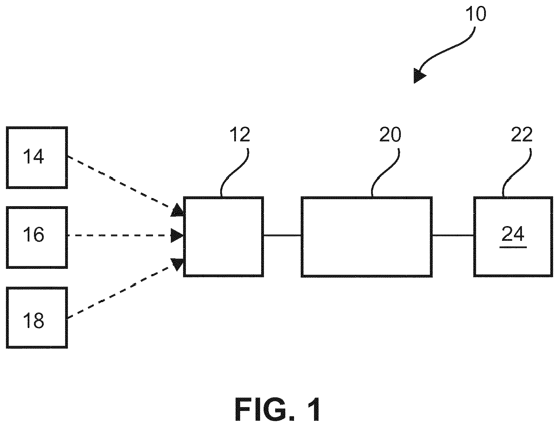

Fig. 1 shows a schematic view of an augmented reality display device 10 for medical equipment. The augmented reality display device 10 comprises a data input unit 12. The data input unit 12 is configured to receive displayed operation parameters 14 of at least one medical appliance. The data input unit 12 is also configured to receive relative location information 16 of at least one medical appliance in relation to the display unit and a viewing direction information 18 of the user. The augmented reality display device 10 also comprises a processing unit 20. The processing unit 20 is configured to detect if at least one of the medical appliances is in the user's field of view based on the relative location information and the viewing direction information. The processing unit 20 is also configured to generate display data based on the operation parameters of the detected medical appliance. The augmented reality display device 10 also comprises a display unit 22. The display unit 22 is configured to project information 24 related to the operation parameters of the medical appliance visible for the user based on the display data if the medical appliance is in the user's field of view. The display unit 22 is also configured to display the information as visible representation overlaid to reality.

In an example, the processing unit 20 is configured to generate duplicate display data based on the operation parameters of the detected medical appliance, and the display unit 22 is configured to project, as the information 24, a duplication of the operation parameters of the medical appliance visible for the user based on the duplicate display data if the medical appliance is in the user's field of view. The display unit 22 is also configured to display the duplication of the operation parameters as visible representation overlaid to reality.

The term "medical equipment" relates to appliances used for medical purposes, such as clinical purposes. For example, the term relates to appliances used for examination of a subject or for interventional procedures.

The term "medical appliance" relates in particular to cathlab and operation room appliances such as subject monitoring equipment, treatment devices, diagnostic devices, imaging devices and other support devices. An example of a treatment or support device are power injectors used to apply for example a controlled dose of a selected substance, e.g. during X-ray imaging. The injection may, for example, be provided in intervals or with a constant flow. Other examples for medical appliances are intravascular ultrasound (IVUS) trolleys or consoles, or fractional flow reserve (FFR) measurement trolleys or consoles, or optical coherence tomography (OCT) measurement trolleys or consoles.

The term "displayed operation parameters" relates to parameters that are indicated or displayed on a medical appliance, e.g. during use of the medical appliance. The parameters thus relate to the operation of the medical appliance. The parameters maybe values that are needed for controlling or adjusting the operation of the medical appliance. The parameters may also be detected values relating to a subject, which values may be measurements taken during operation of the medical appliance.

In an example, values relating to device settings or the respective status are provided before, during and after usage.

The term "relative location information" relates to information about the present positioning or arrangement of a medical appliance in relation to the user of the augmented reality display device.

The term "viewing direction information" relates to a current direction of the user's view to determine which part of the surrounding is actually being seen by the user. As an example, markers are provided attached to the user, e.g. in form of glasses equipped with such markers, that allow the detection of the viewing direction.

The term "field of view" relates to the area actually covered by the user's eyesight. The field of view may be provided as a predetermined angle based on the viewing direction.

The term "duplicate display data" relates to displaying data that is already provided by the medical appliance. The data displayed by the augmented reality display device is thus at least a partly duplication of the data provided by the medical appliance. However, the term "duplicate" relates to a duplication for the user, as other staff members in the room may not see the duplicate display data. Of course, if they are also provided with an example of the augmented reality display device, they will also see - the same or other - duplicate display data.

In an example, the duplicate display data is provided for equipment that is critical for an outcome of the procedure. The equipment can also be referred to as procedure-critical devices.

In an example, the display unit is configured to display the visible representation overlaid to the reality of a live image stream. The live image stream may be provided by display devices such as small monitors, or by the user looking directly at reality through lenses that also provide the duplicate data.

Due to the generated duplicate display, continuous monitoring is supported. Staff members are provided with the information in a facilitate manner, since the information is easy to see, as displayed in the display unit. Further, as only the information of equipment in the user's field of view is presented, the perception of information by the user is facilitated. For example, the important system settings and additional information are rendered and thus provided above or close the device. In an option, depending on the user, i.e. wearer of the augmented reality device, data may be shown at different devices or even different data at the same device.

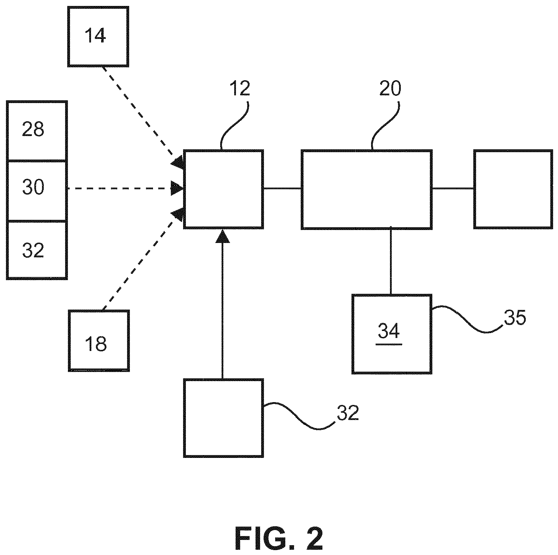

Fig. 2 shows, as an option, an example in which the data input unit 12 is configured to receive, for the relative location information, location information 26 of the at least one medical appliance, position information 28 of the display unit and orientation information 30 of the user. Further, the processing unit is configured to derive the relative location information with respect to the user from this information, i.e. from the location information, the position information and the orientation information. In an option, the viewing direction information 18 is also derived from this information. In another option, the viewing direction information 18 is provided in addition as separate information.

For example, the display unit is provided in form of a wearable device such as glasses, or other devices carried by the user, for example head-worn, or head mounted devices. The viewing direction may also be derived from the positioning information if this information also includes information about a rotational angle of the display unit with respect to the surrounding,

In an example, not further shown in detail, the duplicate visible representation is displayed overlaid to reality depending on the presence of the medical appliance in the user's field of view.

For example, the duplicate visible representation is only shown by the display unit 22 if the medical appliance in the user's field of view. The range covered by the "field of view" can be predetermined and adjusted by the user.

In an example, not shown in detail, the display unit 22 is configured to project the visible representation in the field of view in vicinity of the medical appliance next to the medical appliance. Alternatively, or in addition, the display unit 22 is configured to project the visible representation in the field of view in vicinity of the medical appliance overlaid to the medical appliance. The display unit 22 is configured to also project a device indicator (not shown) for pointing to or highlighting at least one medical appliance for which the visible representation is presented. In an example, an arrow is provided that points from the visible representation towards the respective medical appliance.

In an option, the display unit is provided without the option of projecting the device indicator for pointing to the at least one medical appliance for which the visible representation is presented.

The term "in vicinity" relates to an arrangement or positioning, in which the user can directly identify to which device in his view the presented information is assigned to. In a very crowded environment with a number of devices, the respective arrangement is closer than in a very reduced equipment setup.

The term "next to" relates to an arrangement or positioning, in which, in the field of view of the user, a field with the respective information is in close or direct contact with the visible device to which the information relates to.

The term "overlaid" relates to an arrangement or positioning, in which, in the field of view of the user, the information is at least partly overlapping the visible device to which the information relates to. For example, in the field of view of the user, the information is arranged across the complete device. In an example, in the field of view of the user, the information is arranged within the respective device. In another example, in the field of view of the user, the information extends into an area outside the respective device.

Fig. 2 also shows a further option of an example of the augmented reality display device 10. The data input unit 12 is configured to receive user settings 32 comprising at least information about an assignment to at least one of a plurality of pre-determined user categories 34, for example stored in a data storage 35 connected to the processing unit 20, or stored by the processing unit 20 itself. The user categories 34 can also be provided by other external units or devices. The processing unit 20 is configured to adapt the display data based on the user settings. The option of the user settings entries is provided in addition or alternatively to the other options shown.

Thus, a nurse can be provided with different type of information displayed on the device than a surgeon. The visible duplication is thus provided as a lean duplication or reduced duplication. For example, the user categories provide a first and a second category, such as "physician" and "staff with respectively different duplicate display settings. For example, device and maintenance data is presented to the staff, wherein operational data is presented to the physician, e.g. a surgeon. As an example, in case of a contrast injector, staff members like a nurse are provided with the duplicated data of the remaining time until a new contrast syringe must be placed. The physician is provided with the delivered cumulative contrast volume.

In an example, provided as an option, a plurality of augmented reality display devices is provided. Further, different user categories with assigned duplicate display settings are provided; and the duplicate display data is adjusted based on a selected category with the respectively assigned duplicate display setting. In an example, for different user settings, different data is displayed for the same device. In another example, for different user settings, data is displayed for different devices.

In an example, not further shown, the display unit 22 is configured to present the visible representation of the operation parameters of the medical appliance in a graphical setup that is based on a presentation of the respective operation parameters on a display of the medical appliance. The visible representation is provided as an enlarged duplicate of at least a part of the presentation on the medical appliance.

In an example, a size of the duplicated graphics is rendered independent of the distance, i.e. making it easy to read even is the device is far from the clinician.

In an option, it is provided that the display unit is configured to present the visible representation of the operation parameters of the medical appliance in a graphical setup that is based on a presentation of the respective operation parameters on a display of the medical appliance, but without the enlarged feature.

In another example, the visualization is static. In a still further example, the visualization is non-static and the content changes, given some pre-set threshold like an alarm, or be interacted with using gestures or voice controls. For instance, the graphic could be substantially enlarged when the device is above a certain threshold and return to its original size when "tapped" using gesture controls or even move closer towards the clinician depending on urgency.

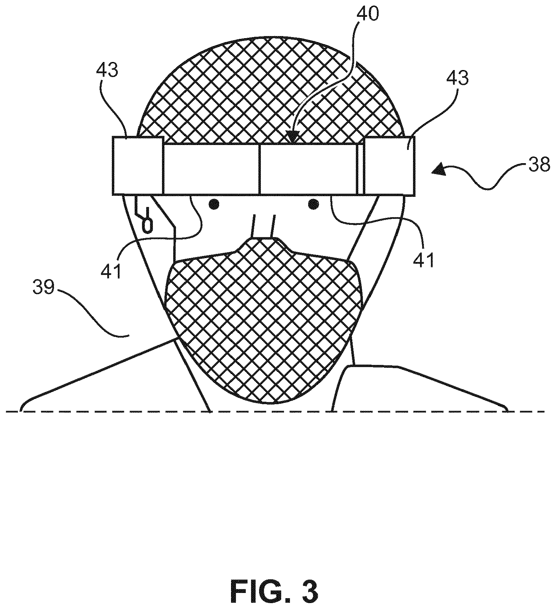

Fig. 3 shows another example of the augmented reality display device 10 where the display is provided as a head mounted display 38. The head mounted display 38 comprises a display device 40, which is configured to allow the user to look through for at least a part of the user's field of view. The display device 40 is also configured to provide the visible representation on the display element within the user's field of view. In the example, the head mounted display 38 is worn by a user 39. The head mounted display 38 may comprise central parts 41 arranged like glasses at least partly within the user's field of view. The central parts 41 may be provided as a left and a right part for each of the user's eyes. Projection units 43 are indicated to arrange the projection e.g. on the lens-type central parts 41 for providing the duplication of the operation parameters of the medical appliance visible for the user such that the visible representation is overlaid to reality as seen by the respective user.

In an option, the display unit is a head mounted display, but without the head mounted display comprising the display device configured i) to allow the user to look through for at least a part of the user's field of view, and ii) to provide the visible representation on the display element within the user's field of view.

In an example, the display device is a transparent element and the visible representation is displayed on the display element, e.g. with a projector element. In another example, the visible representation is displayed in the user's field of view by a hologram technology. In a further example, the head mounted display is a product like the HoloLens (registered trademark) from Microsoft Corporation.

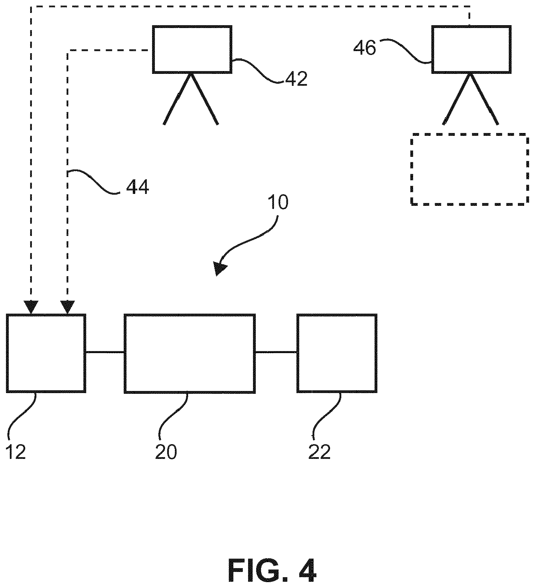

Fig. 4 shows a still further example of the augmented reality display device 10. A viewing direction identifier 42 is provided that is configured to detect a viewing direction of the user in relation to at least one medical appliance and to provide the detected viewing direction 44 to the data input unit 12. Further, in addition, or alternatively, a medical appliance identifier 46 is provided that is configured to identify at least one medical appliance and to provide the displayed operation parameters 14 of the identified at least one medical appliance to the data input unit 12.

In an example, the processing unit 20 is configured to temporarily modify the visible duplication when at least one of the displayed operation parameters reaches a predetermined threshold. The display unit 22 is configured to project the modified visible duplication as an interactive signal to the user. The modified visible duplication thus acts as an interactive signal for the user. The user is provided with a warning signal.

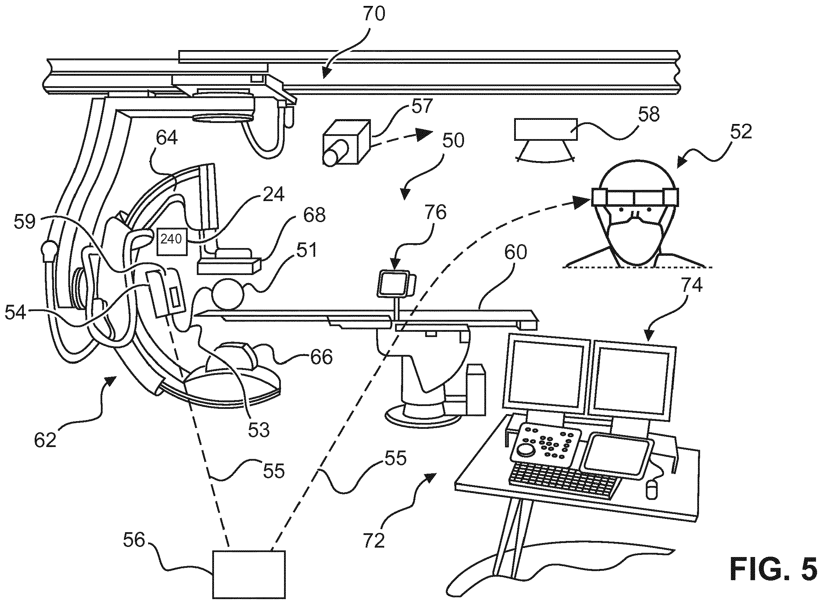

Fig. 5 shows an information system 50 for medical equipment. The system 50 comprises at least one augmented reality display device 52 provided as one of the examples of the augmented reality display device 10 described above. The system 50 further comprises at least one example of the medical appliance 54 with displayed operation parameters. The system 50 also comprises a displayed operation parameters transmitting arrangement 56, which is configured to provide information about the displayed operation parameters from the at least one medical appliance 54 to the data input unit of the at least one augmented reality display device 52.

The "displayed operation parameters transmitting arrangement" can also be referred to as transmitting arrangement for transmission of displayed operation parameters. The displayed operation parameters are provided, namely displayed, by the at least one medical appliance. The "displayed operation parameters transmitting arrangement" is provided to feed the respective information about what is displayed to the processing unit via the data input unit such that the processing unit is capable of generating the duplicated display data based on the transmitted or forwarded information about what is displayed on the device.

In an example (shown as an option), the displayed operation parameters transmitting arrangement 56 comprises a data connection link 55, indicated with two hashed-line arrows, between the at least one medical appliance 54 and the data input unit of the at least one augmented reality display device. In addition, or alternatively, also provided as an option, the displayed operation parameters transmitting arrangement 56 further comprises a camera system 57 providing optical coverage of display areas on the at least one medical appliance 54, e.g. connect to the data input unit via a data connection link.

For the at least one medical appliance 54, a contrast injector is provided, for example. The contrast injector may be provided for injecting contrast agent via a connection 53 to an object 51, for example a subject for examination. The medical appliance 54 is having a display 59, which indicates for example the current flow of 2 ml/s, duration of 8 s, and the available ratio of 240 mg l/ml.

As an example, further equipment of a cathlab is shown. The object is arranged on a support table 60. Further, for X-ray imaging, a C-arm arrangement 62 is provided with a movably supported C-arm 64 and an X-ray source 66 and X-ray detector 68 attached to the ends of the C-arm. A ceiling support 70 with rails and movable carriage is provided for mounting of the C-arm arrangement 92 to a ceiling of the building structure.

A console or control table 72 is shown comprising user interface tools, like a keyboard, and several main displays 74. A further, auxiliary display 76 is provided attached to the patient support 60.

The user 39 is schematically indicated near the patient table, wearing the augmented reality display device 52 provided as the head mounted display. Depending on the arrangement of the medical appliance 54, the display 59 may be hard to identify by the user 39, such as a surgeon. For this purpose, the augmented reality display device provides a visible representation in form of the duplication of the operation parameters of the medical appliance visible only for the user. The duplicate information is shown next to the medical appliance 54 when the medical appliance 54 is in the user's field of view. The display unit of the augmented reality display device 52 is configured to display the duplication of the operation parameters as overlaid to reality augmented reality.

The data connection may be a wire connection or a wireless connection. Data is taken from the medical appliances either directly or in the form of data that is provided to displays of the medical appliances. The data that is present at the medical appliances is then taken to generate the duplicate data for the display unit of the augmented reality display device. In an example, a plurality of medical appliances is connected to a bus-system providing data connection.

The camera system is provided as at least one camera that provides images, i.e. sequences of the displays on the medical appliances. The images are then taken to extract the respective information in order to generate the duplicate data for the display unit of the augmented reality display device. In an example, a plurality of smaller cameras is provided to cover the displays of a plurality of medical appliances.

In an example (also shown as an option), a location tracking device 58 is provided in order to determine a current location of the at least one medical appliance in relation to a subject using one of the at least one augmented reality display devices. The location tracking device 58 comprises at least one of the group of optical cameras, depth cameras, depth sensors, and electromagnetic and optical tracking with markers or tags. In a further option, the location tracking device is provided in order to determine a current location of the at least one medical appliance in relation to a subject using one of the at least one augmented reality display devices, but without the location tracking device comprising at least one of the group of optical camera and electromagnetic tracking with markers or tags.

In an example, a position of the user is tracked to determine relative positions of appliances from an absolute position information.

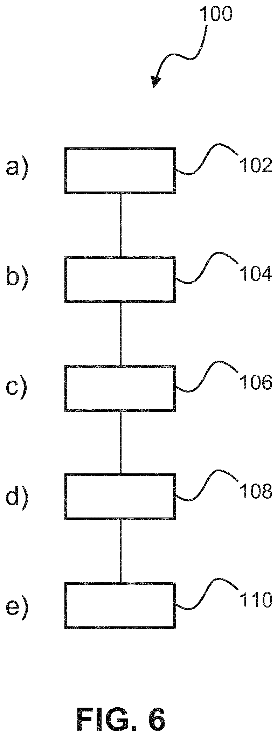

Fig. 6 shows a method 100 for providing operation parameters of medical equipment. The method 100 comprises the following steps. In a first receiving step 102, also referred to as step a), displayed operation parameters of at least one medical appliance is received. In a second receiving step 104, also referred to as step b), relative location information of at least one medical appliance in relation to a display unit of an augmented reality display device for medical equipment, and a viewing direction information of the user are received. The first and the second receiving steps can take place simultaneously or in the order of first receiving step and then second receiving step or vice versa. In a detection step 106, also referred to as step c) it is detected if at least one of the medical appliances is in the user's field of view based on the relative location information and the viewing direction information. In a generating step 108, also referred to as step d) display data is generated based on the operation parameters of the detected medical appliance. In a projection step 110, also referred to as step e), information related to the operation parameters of the medical appliance is projected for the user based on the duplicate display data if the medical appliance is in the user's field of view. The information is displayed as visible representation overlaid to reality of a live image stream as augmented reality.

In an example, step d) comprises generating duplicate display data based on the operation parameters of the detected medical appliance. And step e) comprises projecting a visible duplication of the operation parameters of the medical appliance for the user based on the duplicate display data if the medical appliance is in the user's field of view, wherein the visible duplication is displayed as visible representation overlaid to reality of a live image stream as augmented reality.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An augmented reality display device (10) for medical equipment, comprising:
- a data input unit (12);
- a processing unit (20); and
- a display unit (22);
wherein the data input unit is configured to receive displayed operation parameters (14) of at least one medical appliance; and to receive relative location information (16) of at least one medical appliance in relation to the display unit and a viewing direction information (18) of the user;
wherein the processing unit is configured to detect if at least one of the medical appliances is in the user's field of view based on the relative location information and the viewing direction information; and to generate display data based on the operation parameters of the detected medical appliance; and
wherein the display unit is configured to project information (24) related to the operation parameters of the medical appliance visible for the user based on the display data if the medical appliance is in the user's field of view; and wherein the display unit is configured to display the information as visible representation overlaid to reality.

2. Device according to claim 1, wherein the processing unit is configured to generate duplicate display data based on the operation parameters of the detected medical appliance; and wherein the display unit is configured to project a duplication of at least a part of the operation parameters of the medical appliance visible for the user based on the duplicate display data if the medical appliance is in the user's field of view; and wherein the display unit is configured to display the duplication of the operation parameters as visible representation overlaid to reality.

3. Device according to claim 1 or 2, wherein the data input unit is configured to receive for the relative location information:
- location information (26) of the at least one medical appliance;
- position information (28) of the display unit; and
- orientation information (30) of the user;
wherein the processing unit is configured to derive the relative location information with respect to the user from this information.

4. Device according to claim 1, 2 or 3, wherein the visible representation is displayed overlaid to reality depending on the presence of the medical appliance in the user's field of view; and
wherein the display unit is configured to project the visible representation in the field of view in vicinity of the medical appliance comprising at least one of the group of i) next to the medical appliance and ii) overlaid to the medical appliance; and
wherein the display unit is configured to also project a device indicator for pointing to or highlighting at least one medical appliance for which the visible representation is presented.

5. Device according to one of the preceding claims, wherein the data input unit is configured to receive user settings (32) comprising at least information about an assignment to at least one of a plurality of pre-determined user categories (34); and
wherein the processing unit is configured to adapt the display data based on the user settings.

6. Device according to one of the preceding claims, wherein the display unit is configured to present the visible representation of the operation parameters of the medical appliance in a graphical setup that is based on a presentation of the respective operation parameters on a display of the medical appliance; and
wherein the visible representation is an enlarged duplicate of at least a part of the presentation on the medical appliance.

7. Device according to one of the preceding claims, wherein the display unit is a head mounted display (38); and
wherein the head mounted display comprises a display device (40) which is configured i) to allow the user to look through for at least a part of the user's field of view, and ii) to provide the visible representation on the display element within the user's field of view.

8. Device according to one of the preceding claims, wherein a viewing direction identifier (42) is provided that is configured to detect a viewing direction of the user in relation to at least one medical appliance and to provide the detected viewing direction (44) to the data input unit; and/or
wherein a medical appliance identifier (46) is provided that is configured to identify at least one medical appliance and to provide the displayed operation parameters (48) of the identified at least one medical appliance to the data input unit.

9. Device according to one of the preceding claims, wherein the processing unit is configured to temporarily modify the visible duplication when at least one of the displayed operation parameters reaches a predetermined threshold; and wherein the display unit is configured to project the modified visible duplication as an interactive signal to the user.

10. An information system (50) for medical equipment, the system comprising:
- at least one augmented reality display device (52) according to one of the preceding claims;
- at least one medical appliance (54) with displayed operation parameters; and
- a displayed operation parameters transmitting arrangement (56);
wherein the displayed operation parameters transmitting arrangement is configured to provide information about the displayed operation parameters from the at least one medical appliance to the data input unit of the at least one augmented reality display device.

11. System according to claim 10, wherein the displayed operation parameters transmitting arrangement further comprises:
i) a data connection link between the at least one medical appliance and the data input unit of the at least one augmented reality display device; and/or
ii) a camera system providing optical coverage of display areas on the at least one medical appliance.

12. System according to claim 10 or 11, wherein a location tracking device (58) is provided in order to determine a current location of the at least one medical appliance in relation to a subject using one of the at least one augmented reality display devices; and
wherein the location tracking device comprises at least one of the group of optical cameras, depth cameras, depth sensors, and electromagnetic and optical tracking with markers or tags.

13. A method (100) for providing operation parameters of medical equipment, the method comprising the following steps:
a) receiving (102) displayed operation parameters of at least one medical appliance;
b) receiving (104) relative location information of at least one medical appliance in relation to a display unit of an augmented reality display device for medical equipment and a viewing direction information of the user;
c) detecting (106) if at least one of the medical appliances is in the user's field of view based on the relative location information and the viewing direction information;
d) generating (108) display data based on the operation parameters of the detected medical appliance; and
e) projecting (110) information related to the operation parameters of the medical appliance for the user based on the duplicate display data if the medical appliance is in the user's field of view; wherein the information is displayed as visible representation overlaid to reality of a live image stream as augmented reality.

14. A computer program element for controlling an apparatus according to one of the claims 1 to 12, which program element, when being executed by a processing unit, is adapted to perform the method steps of claim 13.

15. A computer readable medium having stored the program element of claim 14.
